Europäisches Patentamt

(19) European Patent Office

Office européen des brevets

(11) EP 0 753 558 A1

(12) EUROPEAN PATENT APPLICATION

(43) Date of publication:
15.01.1997 Bulletin 1997/03

(51) Int. Cl.6: C09K 3/30, C11D 17/00, A61K 7/00

(21) Application number: 95870086.6

(22) Date of filing: 13.07.1995

(84) Designated Contracting States:
AT BE CH DE DK ES FR GB GR IE IT LI LU NL PT SE

(71) Applicant: THE PROCTER & GAMBLE COMPANY
Cincinnati, Ohio 45202 (US)

(72) Inventor: Etesse, Patrick Jean-François
B-1040 Brussels (BE)

(74) Representative: Canonici, Jean-Jacques et al
Procter & Gamble European Technical Center
N.V.
Temselaan 100
1853 Strombeek-Bever (BE)

(54) Packaged foaming composition

(57) The invention provides a simple aerosol package suitable for use with non-liquifiable propellant gases, which dispenses a light foam (preferably less than 200g, more preferably less than 150g), and which can be completely emptied under the pressure of the propellant gas.

A packaged product comprises a foaming composition and propellant gas packaged within an aerosol container in which at least 0.5 moles/litre of non-liquifiable propellant gas can be charged into a gas-tight measuring vessel, said vessel containing 60% by volume of the foaming detergent composition, when measured at 12 bar at 50°C.

In a second aspect of the invention a method of cleaning textile fabrics is provided using a packaged product as described above, comprising the steps of :

(i) dispensing the foaming composition from the container in the form of a foam having a bulk density less than 250 g/l on to textile fabrics, and
(ii) distributing the foam, either by mechanical agitation, or by hand, over the textile fabrics
(iii) removing the foam residue from the cleaned textile fabrics.

EP 0 753 558 A1

## Description

The present invention relates to a packaged product comprising a foaming composition and a propellant gas packaged in a container. In particular the packaged product is a foam dispensing aerosol container.

It is well known in the aerosol industry that there is a need to move away from fluorocarbon propellants (i.e. chlorofluorocarbon, CFC; hydroflurochlorocarbon, HCFC; and hydrofluorocarbon, HFC) on account of their environmental profile. The replacement choice has mostly been the low molecular weight hydrocarbons such as propane, butane, pentane, hexane, etc., but these are flammable gases which may not always be suitable for use inside confined appliances with potential ignition sources. For these reasons, the industry is seeking a move to more environmentally friendly chemicals.

Replacing the liquid propellants, such as those listed above, by non-liquifiable propellant gases presents new problems. Unlike more conventional liquifiable organic propellant gases, gases such as carbon dioxide and nitrous oxide cannot be liquified at the pressures obtainable in an aerosol container (i.e. typically 10 to 12 bar maximum). As the foaming composition is progressively emptied out of the aerosol canister the carbon dioxide or nitrous oxide in the headspace cannot be replenished as would be the case with liquifiable propellants, and consequently the pressure in the headspace drops. As a result the amount of gas dissolved in the liquid phase decreases progressively as the container is emptied resulting in a progressive increase of the foam density as the container is emptied. Furthermore, if the headspace pressure drops too low it will no longer be possible to dispense a foam from the aerosol container.

One approach to this problem has involved mixtures of liquifiable and non-liquifiable gases, wherein the non-liquifiable gases are highly soluble in the liquifiable gases.

Research Disclosure number RD-170066, published on 10th June 1978, suggests hydrofluorocarbons and hydrochlorofluorocarbons can be employed as co-propellants with carbon dioxide or nitrous oxide to formulate aerosol products such as hair sprays, deodorants, and antiperspirants. Suitable HFCs and CFCs possess a desirable combination of properties including good volatility and an unexpectedly high solubility for carbon dioxide and nitrous oxide. Ostwald solubility coefficients for $CO_2$ and $N_2O$ are greater than 3.5 at 21.1°C (70 °F) for each of the exemplified HFCs and CFCs.

Alternative attempts to address this problem have included the use of microporous structures to adsorb non-liquifiable propellant gas (such as carbon dioxide), thereby providing a "reservoir" of gas from which the headspace pressure can be replenished.

EP-A 0 385 773, published on 5th September 1990, discloses a gas storage system comprising a polymeric material, such as hydrogel, having microvoids functioning as interstitial stores for gas.

DD-A 246 784, published on 17th June 1987, discloses cosmetic and pharmaceutical foam aerosols containing 5-50% of $CO_2$-charged aluminosilicate. It is claimed that filling rates are high, pressure peaks are avoided, and pressure is substantially constant up to complete emptying of the aerosol package.

Foaming compositions which are concentrated in terms of active components, and have a correspondingly low water content may not be suitable for dispensing with carbon dioxide or nitrous oxide because the aerosol container cannot be completely emptied. Furthermore microporous gas adsorbent agents may be undesirable for economic reasons.

It is an aim of the present invention to provide a simple aerosol package suitable for use with non-liquifiable propellant gases, which dispenses a light foam (preferably less than 200g, more preferably less than 150g), and which can be completely emptied under the pressure of the propellant gas.

### Summary of the Invention

According to the invention this object is achieved by a packaged product as specified in claim 1 in which at least 0.5 moles/litre of non-liquifiable propellant gas can be charged into a gas-tight measuring vessel, said vessel containing 60% by volume of the foaming detergent composition, when measured at 12 bar at 50°C.

Preferred non-liquifiable propellant gases are carbon dioxide and nitrous oxide (both with a molecular weight of 44); and preferably the foaming composition has an Ostwald coefficient of at least 1.5, preferably at least 2.5, and more preferably at least 4.

The desired Ostwald coefficients are advantageously achieved by incorporating gas solubilising agents such as aldehydes and ketones into the foaming composition. Particularly preferred gas solubilising agents are acetone, methyl acetate and methylal.

The contribution to total pressure which comes from volatile components or gases other than the non-liquifiable propellant gas is preferably minimised such that the foaming detergent composition has a vapor pressure at 50°C of less than 60 mbar. Correspondingly the total propellant gas partial pressure, is preferably at least 85%, more preferably at least 90%, and most preferably at least 95% of the total pressure inside the container, when measured at 50°C.

In a second aspect of the invention a method of cleaning textile fabrics is provided using a packaged product as described above, comprising the steps of :

(i) dispensing the foaming composition from the container in the form of a foam having a bulk density less than 250 g/l on to textile fabrics, and

(ii) distributing the foam, either by mechanical agitation, or by hand, over the textile fabrics

(iii) removing the foam residue from the cleaned textile fabrics.

In this aspect of the invention water may be used to presoak or rinse, or presoak and rinse, the textile fabrics; it is preferred that the weight ratio of water to dry fabric is less than 1:1.

Detailed Description of the Invention

The amount of gas that can be stored in a container is determined by i) the size of the container; ii) the temperature and pressure inside the container and iii) the thermodynamic equilibrium properties of the products inside the container. In order to satisfy the aerosol directive, a limited choice of container size is available, and certain temperature and pressure requirements must be met.

Consequently the present invention is concerned with the thermodynamic equilibrium properties of the product inside the container, and, in particular with the solubility of the non-liquifiable propellant gas in the foaming composition, and with the partial pressure of the non-liquifiable propellant gas inside the aerosol container.

The term "non-liquifiable propellant gas" as used herein means any gas, or combination of gases which is not in the liquid phase at 50°C and 12 bar. Such gases include carbon dioxide, nitrous oxide, nitrogen, air. Carbon dioxide and nitrous oxide are most preferred.

The solubility of non-liquifiable gases in the foaming composition may be conveniently expressed in terms of the dimentionless Ostwald Coefficient. This is defined as the ratio of the volume of gas absorbed to the volume of the absorbing liquid. For the purposes of measuring Ostwald Coefficients defined herein a constant temperature of 20°C has been used. The solubility of carbon dioxide and nitrous oxide in many organic solvents has been determined. Data given in "The Solubility of Non Electrolytes", by J. Hillebrand, Reinhold Publishing Corporation New York, 3rd Edition, pages 248 to 250.

The total pressure inside the container is the sum of the partial pressures of the gas phase constituents. The gas phase constituents principally comprise the propellant gas, volatile components of the foaming composition and air. The contribution of the partial pressure of air can be advantageously reduced or eliminated by degassing the packaged foaming product prior to gas charging. In order to maximise the amount of propellant gas that can be charged into the container it is preferred that the partial pressure due to the volatile components of the foaming composition is low, in other words volatile components should be present at a low level, if at all, in the foaming composition. Preferably the vapour pressure of the foaming composition is less than 60 mbar when measured at 50°C.

It is preferred that the partial pressure due to the carbon dioxide and/or nitrous oxide propellant gas should be at least 85%, preferably at least 90%, and more preferably at least 95% of the total pressure inside the container, when measured at 50°C. For example, the optimum partial pressure due to the carbon dioxide and/or nitrous oxide propellant gas in an aerosol container packed at 12 bars and 50°C, is at least 11.4 bar.

Foam is a coarse dispersion of gas in a relatively small amount of liquid. The foams of the present invention are a continuous liquid phase comprising a composition, and a dispersed phase comprising a gas. Typically, the gas "bubbles" of the dispersed phase can vary in size from 50 micrometers to several millimetres.

In general, the quality of the foam is determined by assessing various foam quality attributes, such as: 1) the appearance of the foam as it is determined by the uniformity of the bubble size distribution, as well as by the actual bubble sizes, wherein small and uniformly sized bubbles are generally preferred; 2) the thickness of the foam as it is determined by the apparent foam viscosity, wherein a greater apparent foam viscosity is generally preferred; 3) the density of the foam which is preferably less than 250g/l, more preferably less than 150 g/l, and most preferably less than 100 g/l; and 4) the drainage of the liquid from the foam upon standing on a solid surface, wherein slow drainage of the liquid is generally preferred.

Preferred components of the detergent foam will now be described in more detail.

Water-soluble salts of the higher fatty acids, i.e., "soaps", are useful anionic surfactants in the compositions herein. This includes alkali metal soaps such as the sodium, potassium, ethanolamine, ammonium, and alkylammonium salts of higher fatty acids containing from about 8 to about 24 carbon atoms, and preferably from about 12 to about 18 carbon atoms. Soaps can be made by direct saponification of fats and oils or by the neutralization of free fatty acids. Particularly useful are the ethanolamine, sodium and potassium salts of the mixtures of fatty acids derived from coconut oil and tallow, i.e., monoethanolamine, sodium or potassium tallow and coconut soap.

Useful anionic surfactants also include the water-soluble salts, preferably the alkali metal, ethanolamine, ammonium and alkylolammonium salts, of organic sulfuric reaction products having in their molecular structure an alkyl group containing from about 10 to about 20 carbon atoms and a sulfonic acid or sulfuric acid ester group. (Included in the term "alkyl" is the alkyl portion of acyl groups.) Examples of this group of synthetic surfactants are the alkyl sulfates, especially those obtained by sulfating the higher alcohols ($C_8$-$C_{18}$ carbon atoms) such as those produced by reducing the glycerides of tallow or coconut oil; and the alkyl benzene sulfonates in which the alkyl group contains from about 9 to about 15 carbon atoms, in straight or branched chain configuration, e.g., those of the type described in U.S. Pat. Nos. 2,220,099 and 2,477,383; and methyl ester sulphonates. Especially valuable are linear straight chain alkyl benzene sulfonates in which the average number of carbon atoms in the alkyl group is from about 11 to 13, abbreviated as $C_{11}$-$C_{13}$ LAS.

Other anionic surfactants herein are the alkyl glyceryl ether sulfonates, especially those ethers of higher alcohols derived from tallow and coconut oil; coconut oil fatty acid monoglyceride sulfonates and sulfates; salts of alkyl phenol ethylene oxide ether sulfates containing from about 1 to about 10 units of ethylene oxide per molecule and wherein the alkyl groups contain from about 8 to about 12 carbon atoms; and salts of alkyl ethylene oxide ether sulfates containing from about 1 to about 10 units of ethylene oxide per molecule and wherein the alkyl group contains from about 10 to about 20 carbon atoms.

Other useful anionic surfactants herein include the water-soluble salts of esters of alpha-sulfonated fatty acids containing from about 6 to 20 carbon atoms in the fatty acid group and from about 1 to 10 carbon atoms in the ester group; water-soluble salts of 2-acyloxy-alkane-1-sulfonic acids containing from about 2 to 9 carbon atoms in the acyl group and from about 9 to about 23 carbon atoms in the alkane moiety; alkyl ether sulfates containing from about 10 to 20 carbon atoms in the alkyl group and from about 1 to 30 moles of ethylene oxide; watersoluble salts of olefin sulfonates containing from about 12 to 24 carbon atoms; and beta-alkyloxy alkane sulfonates containing from about 1 to 3 carbon atoms in the alkyl group and from about 8 to about 20 carbon atoms in the alkane moiety.

Water-soluble nonionic surfactants are also useful as surfactants in the compositions of the invention. Indeed, preferred processes use anionic/nonionic blends. Such nonionic materials include compounds produced by the condensation of alkylene oxide groups (hydrophilic in nature) with an organic hydrophobic compound, which may be aliphatic or alkyl aromatic in nature. The length of the polyoxyalkylene group which is condensed with any particular hydrophobic group can be readily adjusted to yield a water-soluble compound having the desired degree of balance between hydrophilic and hydrophobic elements.

Suitable nonionic surfactants include the polyethylene oxide condensates of alkyl phenols, e.g., the condensation products of alkyl phenols having an alkyl group containing from about 6 to 16 carbon atoms, in either a straight chain or branched chain configuration, with from about 4 to 25 moles of ethylene oxide per mole of alkyl phenol.

Preferred nonionics are the water-soluble condensation products of aliphatic alcohols containing from 8 to 22 carbon atoms, in either straight chain or branched configuration, with from 1 to 25 moles of ethylene oxide per mole of alcohol, especially 2 to 7 moles of ethylene oxide per mole of alcohol. Particularly preferred are the condensation products of alcohols having an alkyl group containing from about 9 to 15 carbon atoms; and condensation products of propylene glycol with ethylene oxide.

Other preferred nonionics are polyhydroxy fatty acid amides which may be prepared by reacting a fatty acid ester and an N-alkyl polyhydroxy amine. The preferred amine for use in the present invention is N-(R1)-CH2(CH2OH)4-CH2-OH and the preferred ester is a C12-C20 fatty acid methyl ester. Most preferred is the reaction product of N-methyl glucamine (which may be derived from glucose) with C12-C20 fatty acid methyl ester.

Methods of manufacturing polyhydroxy fatty acid amides have been described in WO 9206073, published on 16th April, 1992. This application describes the preparation of polyhydroxy fatty acid amides in the presence of solvents. In a highly preferred embodiment of the invention N-methyl glucamine is reacted with a C12-C20 methyl ester.

Semi-polar nonionic surfactants include water-soluble amine oxides containing one alkyl moiety of from about 10 to 18 carbon atoms and 2 moieties selected from the group consisting of alkyl groups and hydroxyalkyl groups containing from 1 to about 3 carbon atoms; water-soluble phosphine oxides containing one alkyl moiety of about 10 to 18 carbon atoms and 2 moieties selected from the group consisting of alkyl groups and hydroxyalkyl groups containing from about 1 to 3 carbon atoms; and water-soluble sulfoxides containing one alkyl moiety of from about 10 to 18 carbon atoms and a moiety selected from the group consisting of alkyl and hydroxyalkyl moieties of from about 1 to 3 carbon atoms.

Ampholytic surfactants include derivatives of aliphatic or aliphatic derivatives of heterocyclic secondary and tertiary amines in which the aliphatic moiety can be either straight or branched chain and wherein one of the aliphatic substituents contains from about 8 to 18 carbon atoms and at least one aliphatic substituent contains an anionic water-solubilizing group.

Zwitterionic surfactants include derivatives of aliphatic quaternary ammonium phosphonium, and sulfonium compounds in which one of the aliphatic substituents contains from about 8 to 18 carbon atoms.

Useful cationic surfactants include water-soluble quaternary ammonium compounds of the form $R_4R_5R_6R_7N^+X^-$, wherein $R_4$ is alkyl having from 10 to 20, preferably from 12-18 carbon atoms, and $R_5$, $R_6$ and $R_7$ are each $C_1$ to $C_7$ alkyl preferably methyl; $X^-$ is an anion, e.g. chloride. Examples of such trimethyl ammonium compounds include $C_{12-14}$ alkyl trimethyl ammonium chloride and cocalkyl trimethyl ammonium methosulfate.

Other surfactants that may be used in the compositions of the present invention include C10-C18 glycerol ethers, C10-18 alkyl polyglycoside and their corresponding sulphated polyglycosides, alkyl ester sulphonates, and oleoyl sarcosinate.

Enzymes can be included in the foaming compositions herein for a wide variety of fabric laundering purposes, including removal of protein-based, carbohydrate-based, or triglyceride-based stains, for example, and for the prevention of refugee dye transfer, and for fabric restoration. The enzymes to be incorporated include proteases, amylases, lipases, cellulases, and peroxidases, as well as mixtures thereof. Other types of enzymes may also be included. They may be of any suitable origin, such as vegetable, animal, bacterial, fungal and yeast origin. However, their choice is governed by several factors such as pH-activity and/or stability optima, thermostability, stability versus active detergents, builders and so on. In this respect bacterial or fungal enzymes are preferred, such as bacterial amylases and proteases, and fungal cellulases.

Enzymes are normally incorporated at levels sufficient to provide up to about 5 mg by weight, more typically about 0.01 mg to about 3 mg, of active enzyme per gram of the composition. Stated otherwise, the compositions herein will typically comprise from about 0.001% to about 5%, preferably 0.01%-1% by weight of a commercial enzyme preparation. Protease enzymes are usually present in such commercial preparations at levels sufficient to provide from 0.005 to 0.1 Anson units (AU) of activity per gram of composition.

Suitable examples of proteases are the subtilisins which are obtained from particular strains of B. subtilis and B. licheniforms. Another suitable protease is obtained from a strain of Bacillus, having maximum activity throughout the pH range of 8-12, developed and sold by Novo Industries A/S under the registered trade name ESPERASE. The preparation of this enzyme and analogous enzymes is described in British Patent Specification No. 1,243,784 of Novo. Proteolytic enzymes suitable for removing protein-based stains that are commercially available include those sold under the tradenames ALCALASE and SAVINASE by Novo Industries A/S (Denmark) and MAXATASE by International Bio-Synthetics, Inc. (The Netherlands). Other proteases include Protease A (see European Patent Application 130,756, published January 9, 1985) and Protease B (see European Patent Application Serial No. 87303761.8, filed April 28, 1987, and European Patent Application 130,756, Bott et al, published January 9, 1985).

Amylases include, for example, α-amylases described in British Patent Specification No. 1,296,839 (Novo), RAPIDASE, International Bio-Synthetics, Inc. and TERMAMYL, Novo Industries.

The cellulase usable in the present invention include both bacterial or fungal cellulase. Preferably, they will have a pH optimum of between 5 and 9.5. Suitable cellulases are disclosed in U.S. Patent 4,435,307, Barbesgoard et al, issued March 6, 1984, which discloses fungal cellulase produced from Humicola insolens and Humicola strain DSM1800 or a cellulase 212-producing fungus belonging to the genus Aeromonas, and cellulase extracted from the hepatopancreas of a marine mollusk (Dolabella Auricula Solander). suitable cellulases are also disclosed in GB-A-2.075.028; GB-A-2.095.275 and DE-OS-2.247.832. CAREZYME (Novo) is especially useful.

Suitable lipase enzymes for detergent usage include those produced by microorganisms of the Pseudomonas group, such as Pseudomonas stutzeri ATCC 19.154, as disclosed in British Patent 1,372,034. See also lipases in Japanese Patent Application 53,20487, laid open to public inspection on February 24, 1978. This lipase is available from Amano Pharmaceutical Co. Ltd., Nagoya, Japan, under the trade name Lipase P "Amano," hereinafter referred to as "Amano-P." Other commercial lipases include Amano-CES, lipases ex Chromobacter viscosum, e.g. Chromobacter viscosum var. lipolyticum NRRLB 3673, commercially available from Toyo Jozo Co., Tagata, Japan; and further Chromobacter viscosum lipases from U.S. Biochemical Corp., U.S.A. and Disoynth Co., The Netherlands, and lipases ex Pseudomonas gladioli. The LIPOLASE enzyme derived from Humicola lanuginosa and commercially available from Novo (see also EPO 341,947) is a preferred lipase for use herein.

Peroxidase enzymes are used in combination with oxygen sources, e.g., percarbonate, perborate, persulfate, hydrogen peroxide, etc. They are used for "solution bleaching," i.e. to prevent transfer of dyes or pigments removed from substrates during wash operations to other substrates in the wash solution. Peroxidase enzymes are known in the art, and include, for example, horseradish peroxidase, ligninase, and haloperoxidase such as chloro- and bromo-peroxidase. Peroxidase-containing detergent compositions are disclosed, for example, in PCT International Application WO 89/099813, published October 19, 1989, by O. Kirk, assigned to Novo Industries A/S.

A wide range of enzyme materials and means for their incorporation into synthetic detergent compositions are also disclosed in U.S. Patent 3,553,139, issued January 5, 1971 to McCarty et al. Enzymes are further disclosed in U.S. Patent 4,101,457, Place et al, issued July 18, 1978, and in U.S. Patent 4,507,219, Hughes, issued March 26, 1985, both. Enzyme materials useful for liquid detergent formulations, and their incorporation into such formulations, are disclosed in U.S. Patent 4,261,868, Hora et al, issued April 14, 1981. Enzymes for use in detergents can be stabilized by various techniques. Enzyme stabilization techniques are disclosed and exemplified in U.S. Patent 3,600,319, issued August 17, 1971 to Gedge, et al, and European Patent Application Publication No. 0 199 405, Application No. 86200586.5, published October 29, 1986, Venegas. Enzyme stabilization systems are also described, for example, in U.S. Patent 3,519,570.

The foam of the present invention can contain neutral or alkaline salts which have a pH in solution of seven or greater, and can be either organic or inorganic in nature. The builder salt assists in providing the desired density and bulk to the detergent granules herein. While some of the salts are inert, many of them also function as detergency builder materials in the laundering solution.

Examples of neutral water-soluble salts include the alkali metal, ethanolamine, ammonium or substituted ammonium chlorides, fluorides and sulfates. The sodium, ethanolamine and ammonium salts of the above are preferred. Citric acid and, in general, any other organic or inorganic acid may be incorporated into the present invention.

Other useful water-soluble salts include the compounds commonly known as detergent builder materials. Builders are generally selected from the various water-soluble, alkali metal, ethanolamine, ammonium or substituted ammonium phosphates, polyphosphates, phosphonates, polyphosphonates, carbonates, silicates, borates, and polyhydroxysulfonates. Preferred are the sodium, ethanolamine and ammonium salts of the above.

Specific examples of inorganic phosphate builders are sodium and potassium tripolyphosphate, pyrophosphate, polymeric metaphosphate having a degree of polymerization of from about 6 to 21, and orthophosphate. Examples of polyphosphonate builders are the salts of ethylene diphosphonic acid, the salts of ethane 1-hydroxy-1,1-diphosphonic acid and the salts of ethane, 1,1,2-triphosphonic acid. Other phosphorus builder compounds are disclosed in U.S. Pat. Nos. 3,159,581; 3,213,030; 3,422,021; 3,422,137; 3,400,176 and 3,400,148, incorporated herein by reference. In general, however, phosphates are preferably avoided for environmental reasons.

Examples of nonphosphorus, inorganic builders are sodium and potassium carbonate, bicarbonate, sesquicarbonate, tetraborate decahydrate, and silicate having a molar ratio of $SiO_2$ to alkali metal oxide of from about 0.5 to about 4.0, preferably from about 1.0 to about 2.4.

Foam stabilising agents may also be employed in the compositions of the present invention. Especially preferred are alyphatic alcohols such as straight chain saturated alcohols of 12 to 18 carbon atoms e.g. cetyl alcohol, stearyl alcohol, myristyl alcohol and mixtures thereof. Polymers including polyvinylpyrrolidone, polyvinyl alcohol, polyacrylamide, polypeptides, polysaccharides, cellulose derivatives; and also natural and synthetic gums and resins such as guar gum, xanthan gum, carageenan, sodium alginate and caseinate may also be used in the present invention.

Test Method

A. Determination of gas amount

For the purposes of defining the present invention the amount of gas that can be charged into a sealed measuring vessel containing 60% by volume of the foaming composition must be determined at 12 bar and 50°C. Whilst any measuring vessel may be used for this determination, (provided that it is rigid enough to withstand the internal pressure without deformation) it is convenient to use a standard 405 ml aerosol container.

1. The container is filled to 60% of its effective real capacity (i.e. 60% of 400 ml = 240 ml)

2. A measured weight of gas is added to the container so that the foaming composition is saturated, and the headspace is filled with pressurised gas at 12 bar and 50°C. The total weight of gas charged is recorded.

3. The amount of gas added in moles/litre is calculated as:

(moles/litre) = Wt. of gas charged (g) / (Molecular Weight of gas x Total Volume of Measuring Vessel (l))
= Wt. of gas charged (g) / (Molecular Weight of gas x 0.4)

B. Determination of Ostwald Coefficient (at 20°C)

(1) Fill a glass pressure vessel to its nominal volume.

(2) Seal the vessel and degas the foaming composition by pulling a vacuum on top of the liquid.

(3) After degassing of the vessel and its contents, the following variables are measured:

- the pressure inside the degassed vessel which is the foaming composition vapor pressure;
- the volume of the degassed liquid and ;
- the total weight of the package and its content M0.

(4) Pressurize the vessel to its nominal pressure and wait for equilibrium to be reached. Equilibrium is reached when no pressure drop is observed vs. time.

(5) Once equilibrium has been reached, the following variables are measured:

- the volume of the head space,
- the gas partial pressure = total pressure - foaming composition vapor pressure.
- the total amount of gas inside the can which is the weight increase since M0. At this stage, it is convenient to convert the gas amount from weight to number of moles by dividing it by the molecular weight of $CO_2$ or $N_2O$ which are incidentally the same i.e. 44g.

(6) The number of gas moles inside the head space is calculated using the ideal gas law (valid for pressures below 10 bars) and is discounted to the total amount inside the vessel. This is the amount of gas in the liquid phase.

(7) The amount of gas in the liquid phase can now be converted to the volume of gas absorbed in the liquid phase (by calculating the volume at partial pressure P(gas) and 20°C using the ideal gas law) and the Ostwald coefficient is found by dividing this volume by the degassed liquid volume.

Examples

All components are expressed in % by weight.
Nonionic surfactant is C12-C14 fatty alcohol ethoxylated with an average of 7 ether groups per mole.
Fatty acid amide is polyhydroxy fatty (C12-C14) acid amide Alkyl sulphate is expressed in terms of C12-C14 alkyl sulphuric acid.
Alkyl ether sulphate is expressed in terms of C12-C14 alkyl sulphuric acid, with an average of 3 ether groups per mole.

| | Ex. 1 | Ex. 2 | Ex. 3 | Comparative Example A |
|---|---|---|---|---|
| Methylal | 7 | 15 | 27 | - |
| Water | 23.25 | 21.25 | 18.25 | 25 |
| Propane-1,2-diol | 10.23 | 9.35 | 8.03 | 11 |
| Ethanol | 0.93 | 0.85 | 0.73 | 1 |
| Nonionic surfactant | 13.95 | 12.75 | 10.95 | 15 |
| Fatty acid amide | 5.58 | 5.1 | 4.38 | 6 |
| Alkyl sulphate | 10.23 | 9.35 | 8.03 | 11 |
| Alkyl ether sulphate | 10.23 | 9.35 | 8.03 | 11 |
| Fatty acid | 12.09 | 11.05 | 9.49 | 13 |
| NaOH | 1.86 | 1.7 | 1.46 | 2 |
| Monoethanolamine | 3.72 | 3.4 | 2.92 | 4 |
| Enzymes | 0.93 | 0.85 | 0.73 | 1 |

The compositions (240ml in each case) of Examples 1 to 3, and Comparative Example A were charged into aerosol containers having a nominal capacity of 405ml (effective real capacity of 400ml). Each container was then charged with carbon dioxide until the compositions were saturated. The amount of carbon dioxide charged into the container was such that the total pressure would reach 12 bar at 50°C.

| | Ex. 1 | Ex. 2 | Ex. 3 | Comparative Example A |
|---|---|---|---|---|
| Ostwald Coefficient at 20°C | 1.74 | 2.5 | 4.1 | 0.82 |
| Amount of gas at 12 bar and 50°C (mole/litre) | 0.51 | 0.68 | 1.02 | 0.38 |
| Foam density (grams/litre) | 167 | 108 | n/a | 223 |

The foam density in Example 3 was not measurable because of the very rapid rate of collapse of the foam.
Comparative Example A was repeated using nitrous oxide instead of carbon dioxide. The Ostwald Coefficient was found to be 0.94, and less than 0.5 mole/litre was charged into the container when the composition was saturated with gas at 12 bar and 50°C. The foam density was 205 g/l.

**Claims**

1. A packaged product comprising

   (i) a foaming composition;
   (ii) propellant gas; and
   (iii) a container having a dispensing means;

   wherein the foaming composition and the propellant gas are packaged within the container in the absence of chlorofluorocarbon, hydroflurochlorocarbon, and hydrofluorocarbon, and in the absence of solid gas-adsorbent microporous structures,
   characterised in that at least 0.5 moles/litre of non-liquifiable propellant gas can be charged into a gas-tight measuring vessel, said vessel containing 60% by volume of the foaming detergent composition, when measured at 12 bar at 50°C.

2. A packaged product according to claim 1 wherein that the foaming composition has an Ostwald Coefficient at 20°C of at least 1.5 in respect of the propellant gas, the propellant gas being selected from the group consisting of carbon dioxide, nitrous oxide, or mixtures thereof.

3. A packaged product according to claim 2 wherein the foaming composition has an Ostwald Coefficient at 20°C of at least 2.5, preferably at least 4.

4. A packaged product according to any of claims 1 to 3 wherein the foaming composition comprises a gas solubilising agent selected from the group consisting of ketones, aldehydes, or mixtures thereof.

5. A packaged product according to any of claims 1 to 3 wherein the foaming composition comprises a gas solubilising agent selected from the group consisting of acetone, methyl acetate, methylal, or mixtures thereof.

6. A packaged product according to claim 1 wherein the foaming detergent composition has a vapor pressure at 50°C of less than 60 mbar.

7. A packaged product according to claim 2 having a total propellant gas partial pressure, of at least 85%, preferably at least 90%, and more preferably at least 95% of the total pressure inside the container, when measured at 50°C.

8. A method of cleaning textile fabrics using a packaged product according to any of the previous claims comprising the steps of :

   (i) dispensing the foaming composition from the container in the form of a foam having a bulk density less than 250 g/l on to textile fabrics, and
   (ii) distributing the foam, either by mechanical agitation, or by hand, over the textile fabrics
   (iii) removing the foam residue from the cleaned textile fabrics.

9. A method of cleaning textile fabrics according to claim 5 whereby water is used to presoak or rinse, or presoak and rinse, the textile fabrics, wherein the weight ratio of water to dry fabric is less than 1:1.

European Patent Office

**EUROPEAN SEARCH REPORT**

Application Number

EP 95 87 0086

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| E | EP-A-0 677 577 (PROCTER AND GAMBLE) * abstract; claims 1,2,5,9 * | 1-9 | C09K3/30 C11D17/00 A61K7/00 |
| X | EP-A-0 586 295 (NLN) * abstract; claims 1,7-9 * * page 3, line 23; claim 38 * | 1-9 | |
| A | DATABASE WPI Week 8906 Derwent Publications Ltd., London, GB; AN 89-042692 & JP-A-63 314 299 (PEGION KK) , 22 December 1988 * abstract * | | |
| A | FR-A-2 477 414 (L'ORÉAL) * claims 1,2,14 * | 1-9 | |
| A | WO-A-93 09761 (RICHARDSON-VICKS) * abstract; claims 1-8 * | 1-9 | |
| A | DE-A-35 30 915 (L'ORÉAL) * abstract; claims 1-5,14 * | 1-9 | TECHNICAL FIELDS SEARCHED (Int.Cl.6) C09K C11D A61K |
| A | FR-A-2 345 996 (GILLETTE COMPANY) * claims 1-4; table 1 * | 1-9 | |

The present search report has been drawn up for all claims

| Place of search | Date of completion of the search | Examiner |
|---|---|---|
| THE HAGUE | 20 December 1995 | Nicolas, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)